# EUROPEAN PATENT APPLICATION

(11) **EP 4 369 686 A1**
(43) Date of publication of application: **15.05.2024**
(21) Application number: 22206443.8
(22) Date of filing: 09.11.2022
(51) Int. Cl.: H04L 67/00, G16H 40/40, H04L 9/40, H04L 67/02, H04L 67/12, H04W 4/80

(54) **IMPLANT COMMUNICATION SYSTEM AND METHOD FOR COMMUNICATING WITH AN IMPLANTABLE MEDICAL DEVICE**

(71) Applicant: BIOTRONIK SE & Co. KG, 12359 Berlin (DE)
(72) Inventor: Wunderlich, Hanno, 13355 Berlin (DE)
(74) Representative: Biotronik Corporate Services SE

(57) **Abstract**

The invention relates to an implant communication system (1), comprising a front-end communication unit (10) comprising a first communication interface (12) configured to read out first data (D1) from an implantable medical device (14) and to write second data (D2) to the implantable medical device (14), the front-end communication unit (10) further comprising a control unit (16) communicatively connected to the first communication interface (12), the control unit (16) being configured to transfer the first data (D1) to a back-end communication unit (18) and to transfer the second data (D2) via the first communication interface (12) to the implantable medical device (14), and the back-end communication unit (18) comprising a programmer (20) configured to program the implantable medical device (14) through a real-time, bidirectional communication with the implantable medical device (14) via the control unit (16) and the first communication interface (12) of the front-end communication unit (10). Furthermore, the invention relates to a computer implemented method for communicating with an implantable medical device (14) (14) and a computer program.

## Description

The invention relates to an implant communication system. Furthermore, the invention relates to a computer implemented method for communicating with an implantable medical device.

Conventional implant systems comprise a programmer capable of reading out data from and writing data to an implantable medical device. Such proprietary programming devices can also be used solely for reading out data from the implantable medical device.

If a patient wearing an implantable medical device is admitted to a hospital and/or medical practice due to an acute medical condition data is read out from the implantable medical device by means of said programmer by a trained expert and/or medical practitioner in order to evaluate medical parameters of the patient recorded by the implantable medical device as well as technical parameters of the implantable medical device in order to evaluate if the implantable medical device is operating normally or whether a technical issue is present.

Programmers however are not universally available at all medical facilities such as hospitals and/or medical practices. Furthermore, such programmers may be difficult to use for untrained medical staff, acceptance of such devices is generally limited. In addition, programming devices are equipped with many functions which, if used incorrectly, can adversely affect the health of the patient / implant carrier.

US 2020/0093431 A1 discloses alert implants that comprise a medical device and an implantable reporting processor, where one example of such a medical device includes a component for a total knee arthroplasty such as a tibial extension, a femoral component for hip replacements, a breast implant, a distal rod for arm or leg breakage repair, a scoliosis rod, a dynamic hip screw, a spinal interbody spacer, and tooling and methods that may be used to form the alert implant, and uses of such alert implants in the health maintenance of patients who receive the implant.

The above-mentioned solution thus allows to monitor a host activity, store measurements and output data. However, there is a currently unmet need to simplify adjusting a programming and/or setting of an implantable medical device in response to data recorded by the implantable medical device necessitating said adjustment of programming and/or settings of the implantable medical device.

It is therefore an object of the present invention to provide an improved implant communication system that provides a simplified way of adjusting a programming and/or setting of an implantable medical device in response to data recorded by the implantable medical device.

The object is solved by an implant communication system having the features of claim 1.

Furthermore, the object is solved by a computer implemented method for communicating with an implantable medical device having the features of claim 14.

In addition, the object is solved by a computer program with program code to perform the method according to the present invention when the computer program is executed on a computer having the features of claim 15.

Further developments and advantageous embodiments are defined in the dependent claims.

The present invention provides an implant communication system, comprising a front-end communication unit comprising a first communication interface configured to read out first data from an implantable medical device and to write second data to the implantable medical device, the front-end communication unit further comprising a control unit communicatively connected to the first communication interface, the control unit being configured to transfer the first data to a back-end communication unit and to transfer the second data via the first communication interface to the implantable medical device.

Furthermore, the implant communication system comprises a back-end communication unit comprising a programmer configured to program the implantable medical device through a real-time, bidirectional communication with the implantable medical device via the control unit and the first communication interface of the front-end communication unit.

Moreover, the present invention provides a computer implemented method for communicating with an implantable medical device. The method comprises providing a front-end communication unit comprising a first communication interface configured to read out first data from an implantable medical device and to write second data to the implantable medical device.

In addition, the method comprises providing a control unit comprised by the front-end communication unit, the control unit being communicatively connected to the first communication interface and transferring the first data to a back-end communication unit by means of the control unit.

The method furthermore comprises transferring the second data via the first communication interface to the implantable medical device, and programming the implantable medical device through a real-time, bidirectional communication with the implantable medical device via the control unit and the first communication interface of the front-end communication unit by means of a programmer comprised by the back-end communication unit.

Furthermore, the present invention provides a computer program with program code to perform the method of the present invention when the computer program is executed on a computer.

An idea of the present invention is to provide an implant communication system that enables clinics and physicians in private practice with a small number of follow-up visits per year, for which the provision of a local programmers is not cost-efficient, to have follow-up visits performed by experts with a programmer.

The physician operating the programmer advantageously does not need to be on-site with the patient to perform the programming. This can also be very helpful in satellite clinics when the local staff is not sufficiently trained in programming a particular type of implant.

In addition to programming, this solution also allows tests to be performed and more extensive data to be read out that is ordinarily not queried as part of a regular data read-outs.

The second data can comprise configuration data and/or programming data of the implantable medical device such as a stimulus threshold, a pacing rate, a data recording rate and/or the enablement or disablement of an MRI-mode.

The implantable medical device may be formed by a purely therapeutic implant, an implant with therapeutic and diagnostic functions and/or an implant with therapeutic and diagnostic functions.

An example of a purely therapeutic implant / implantable medical device is, e.g. a stimulator / electrode for deep brain stimulation. The therapy consists of the delivery of pulse trains without collecting diagnostic data from the stimulator.

An example of a purely diagnostic implant is, e.g. a cardiac rhythm monitor. The diagnostic function consists of continuous recording of the patient's ECG and automatic evaluation of abnormalities of the heart rhythm. If such are detected, an ECG recording is stored and typically automatically transmitted to a remote monitoring system.

An example of an implant with therapeutic and diagnostic functions is e.g. a cardiac pacemaker. The pacemaker is typically implanted subcutaneously in the upper right thoracic region and the electrode is placed in the patient's heart via a large vein. The therapeutic function consists of delivering stimulation pulses to trigger a cardiac action, provided there is no spontaneous cardiac action in the patient. The diagnostic function consists, for example, in the continuous recording of the patient's ECG and automatic evaluation of abnormalities of the heart rhythm. If such are detected, an ECG recording is stored and typically automatically transmitted to a remote monitoring system.

According to an aspect of the invention, the programmer is configured to transmit a first control signal and/or the first data to the control unit of the front-end communication unit, wherein the control unit transmits the first control signal and/or the first data to the first communication interface, and wherein the first communication interface transmits the first control signal and/or the first data to the implantable medical device.

The control signal of the programmer is thus relayed to the first communication interface via the control unit in order to perform a real-time data exchange with the implantable medical device.

According to an aspect of the invention, the first communication interface of the front-end communication unit is configured to transmit a second control signal and/or the second data read out from the implantable medical device to the control unit of the front-end communication unit, and wherein the control unit is configured to transmit the second control signal and/or the second data to the programmer. Said real-time data exchange is thus bi-directional.

According to an aspect of the invention, the control unit and the programmer are configured to communicate through a peer-to-peer network connection or a client-server network connection, in which the control unit comprises a client application and the programmer comprises a server application. The programmer thus provides versatile connectivity options adaptable to a specific implementation.

According to an aspect of the invention, the programmer is configured to initiate the network connection with the control unit by inputting, in particular by a user of the programmer, a session key generated by the control unit. In doing so, a secure operation of the programmer by an authorized user can be performed.

According to an aspect of the invention, the programmer is configured to establish a connection with the control unit via a cloud server. This offers the possibility that the location of the programmer can be flexible since only an IP-connection to the cloud server is required.

According to an aspect of the invention, if the session key input into the programmer matches the session key generated by the control unit, wherein the programmer and the control unit have a network connection to the cloud server, the programmer and the control unit are configured to establish an encrypted connection with each other via the cloud server. Encrypting the communication thus provides an additional layer of security.

According to an aspect of the invention, the implant communication system comprises or uses a time server. The time server is (only) needed and/or configured to provide the correct time to the front-end communication unit (if needed). A correct time is a requirement for a functioning (encrypted) connection between two communication partners (such as front-end communication unit and back-end communication unit).

According to an aspect of the invention, the communications units (i.e. the front-end communication unit and the back-end communication unit) are configured to establish a secure communication link between each other using the session key or another key. From the establishment of a session (the secure communication link) by means of the session key or another key, a shared secret is to emerge/create, which is used to encrypt/decrypt data (e.g. the first data and/or the second data).

According to an aspect of the invention, the programmer is a standalone medical programming device and/or an app on a smartphone or tablet computing device, said programmer further having Bluetooth-, WLAN- und/or LAN-based connectivity. The type of device used can thus be chosen in accordance with the specific technical implementation of the implant communication system.

According to an aspect of the invention, if the network connection between the control unit and the programmer is established, the programmer is configured to redirect the communication of the programmer to the first communication interface to a communication between the programmer and the control unit. During remote usage of the programmer, it therefore communicates directly with the control unit as opposed to the first communicates interface/programming head during ordinary non-remote operation of the programmer.

According to an aspect of the invention, upon establishment of the communication between the programmer and the control unit, the first communication interface is placeable in proximity to the implantable medical device in order to read out the first data, in particular a model type, a firmware version and/or current setting data, in particular a current programming, from the implantable medical device and/or to write the second data to the implantable medical device. The second data is thus advantageously based on the first data, thus ensuring that the second data is sent to the correct implantable medical device using the unique identifiers contained in the first data.

According to an aspect of the invention, the control unit of the front-end communication unit is configured to check an integrity of the second data for the implantable medical device and whether the second data is intended for the currently interrogated implantable medical device. This additional level of security ensures that the second data can only be used to communicate with a matching implantable medical device.

According to an aspect of the invention, if the integrity of the second data and a correctness of the currently interrogated implantable medical device are confirmed, the first communication interface is configured to transfer the second data to the implantable medical device, wherein after programming the implantable medical device, the front-end communication unit is configured to send a confirmation message to the programmer. The data communication process is hence indicated to be completed.

The herein described features of the implant communication system are also disclosed for the computer implemented method for communicating with an implantable medical device and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a diagram of an implant communication system according to a preferred embodiment of the invention; and
- Fig. 2: shows a flowchart of a computer implemented method for communicating with an implantable medical device according to the preferred embodiment of the invention.

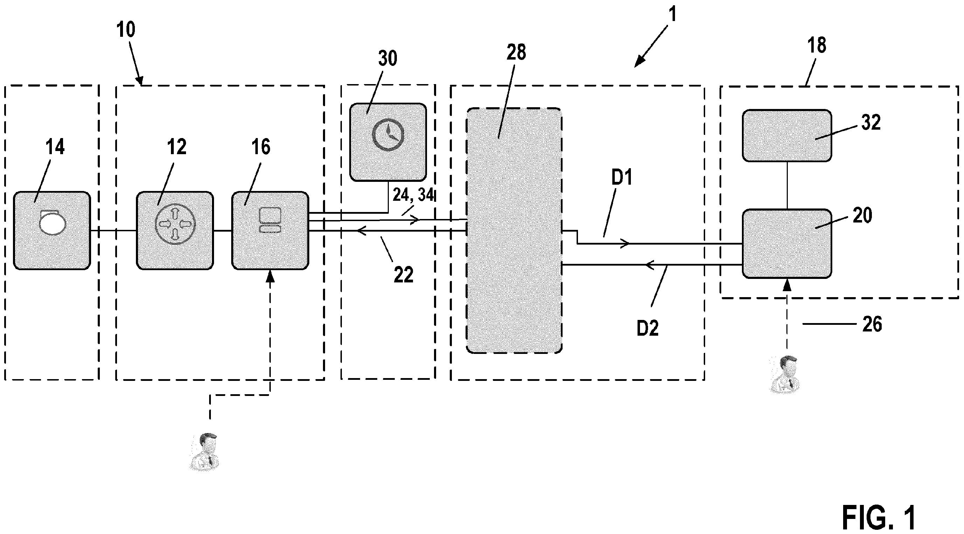

The implant communication system 1 of Fig. 1 comprises a front-end communication unit 10 comprising a first communication interface 12 configured to read out first data D1 from an implantable medical device 14 and to write second data D2 to the implantable medical device 14. The front-end communication unit 10 further comprises a control unit 16 communicatively connected to the first communication interface 12, the control unit 16 being configured to transfer the first data D1 to a back-end communication unit 18 and to transfer the second data D2 via the first communication interface 12 to the implantable medical device 14.

In addition, the back-end communication unit 18 comprises a programmer 20 configured to program the implantable medical device 14 through a real-time, bidirectional communication with the implantable medical device 14 via the control unit 16 and the first communication interface 12 of the front-end communication unit 10.

The programmer 20 is configured to transmit a first control signal 22 and/or the first data D1 to the control unit 16 of the front-end communication unit 10. Furthermore, the control unit 16 transmits the first control signal 22 and/or the first data D1 to the first communication interface 12. The first communication interface 12 moreover transmits the first control signal 22 and/or the first data D1 to the implantable medical device 14.

The first communication interface 12 of the front-end communication unit 10 is further configured to transmit a second control signal 24 and/or the second data D2 read out from the implantable medical device 14 to the control unit 16 of the front-end communication unit 10. Moreover, the control unit 16 is configured to transmit the second control signal 24 and/or the second data D2 to the programmer 20.

The control unit 16 and the programmer 20 are further configured to communicate through a peer-to-peer network connection or a client-server network connection in which the control unit 16 comprises a client application and the programmer 20 comprises a server application.

The programmer 20 is moreover configured to initiate the network connection with the control unit 16 by inputting, in particular by a user of the programmer 20, a session key 26 generated by the control unit 16. Furthermore, the programmer 20 is configured to establish a connection with the control unit 16 via a cloud server 28.

If the session key 26 input into the programmer 20 matches the session key 26 generated by the control unit 16, wherein the programmer 20 and the control unit 16 have a network connection to the cloud server 28, the programmer 20 and the control unit 16 are configured to establish an encrypted connection with each other via the cloud server 28.

A time server 30 is further configured to provide the correct time to the front-end communication unit 10 (if needed).

The communications units 10, 18 are configured to establish a secure communication link between each other using the session key. The session key is needed to encrypt/decrypt the first data D1 and/or second data D2. The back-end communication unit 18 is configured to decrypt the (encrypted) first data D1 and to encrypt the second data D2. Furthermore, the front-end communication unit 10 is configured to encrypt the first data D1 and to decrypt the (encrypted) second data D2.

Furthermore, the programmer 20 is a standalone medical programming device and/or an app on a smartphone or tablet computing device, said programmer 20 further having Bluetooth, WLAN- and/or LAN-based connectivity.

If the network connection between the control unit 16 and the programmer 20 is established, the programmer 20 is configured to redirect the communication of the programmer 20 to the first communication interface 12 to a communication between the programmer 20 and the control unit 16.

Upon establishment of the communication between the programmer 20 and the control unit 16, the first communication interface 12 is placeable in proximity to the implantable medical device 14 in order to read out the first data D1, in particular a model type, a firmware version and/or current setting data, in particular a current programming, from the implantable medical device 14 and/or to write the second data D2 to the implantable medical device 14.

A local user preferably operates the control unit 16. Placing the first communication interface 12, which is the programming head of the front-end communication unit 10, near the patient implantable medical device 14, the data transfer to the implantable medical device 14 and vice versa can be initiated. An authorized user such as a medical practitioner operates the programmer 20.

The control unit 16 of the front-end communication unit 10 is further configured to check an integrity of the second data for the implantable medical device 14 and whether the second data is intended for the currently interrogated implantable medical device 14.

If the integrity of the second data and a correctness of the currently interrogated implantable medical device 14 are confirmed, the first communication interface 12 is configured to transfer the second data D2 to the implantable medical device 14, wherein after programming the implantable medical device 14, the front-end communication unit 10 is configured to send a confirmation message 34 to the programmer 20.

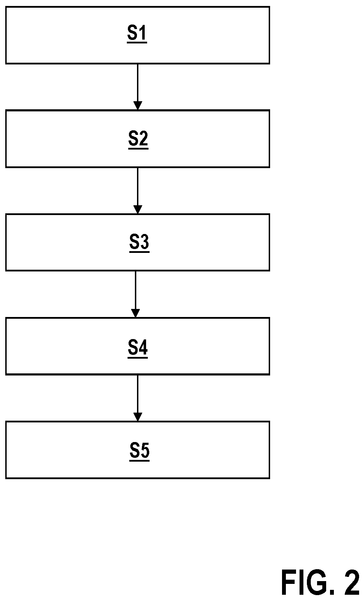

Fig. 2 shows a flowchart of a computer implemented method for communicating with an implantable medical device 14 according to the preferred embodiment of the invention.

The method comprises providing S1 a front-end communication unit 10 comprising a first communication interface 12 configured to read out first data D1 from an implantable medical device 14 and to write second data D2 to the implantable medical device 14.

In addition, the method comprises providing S2 a control unit 16 comprised by the front-end communication unit 10, the control unit 16 being communicatively connected to the first communication interface 12 and transferring S3 the first data D1 to a back-end communication unit 18 by means of the control unit 16.

The method moreover comprises transferring S4 the second data D2 via the first communication interface 12 to the implantable medical device 14, and programming S5 the implantable medical device 14 through a real-time, bidirectional communication with the implantable medical device 14 via the control unit 16 and the first communication interface 12 of the front-end communication unit 10 by means of a programmer 20 comprised by the back-end communication unit 18.

Although specific embodiments have been illustrated and described herein, it will be understood by those skilled in the art that a variety of alternative and/or equivalent implementations exist. It should be noted that the exemplary embodiment or exemplary embodiments are examples only and are not intended to limit the scope, applicability, or configuration in any way.

Rather, the foregoing summary and detailed description provides those skilled in the art with convenient guidance for implementing at least one exemplary embodiment, it being understood that various changes in the functional scope and arrangement of the elements may be made without departing from the scope of the appended claims and their legal equivalents.

In general, this application intends to cover modifications or adaptations or variations of the embodiments disclosed herein. For example, an order of the method steps may be modified. The method may further be carried out sequentially or in parallel, at least in part.

### Reference Signs

- 1: implant communication system
- 10: front-end communication unit
- 12: first communication interface
- 14: implantable medical device
- 16: control unit
- 18: back-end communication unit
- 20: programmer
- 22: first control signal
- 24: second control signal
- 26: session key
- 28: cloud server
- 30: time server
- 32: certificate and key management system
- 34: confirmation message
- D1: first data
- D2: second data
- S1-S5: method steps

## Claims

1. Implant communication system (1), comprising:
a front-end communication unit (10) comprising a first communication interface (12) configured to read out first data (D1) from an implantable medical device (14) and to write second data (D2) to the implantable medical device (14), the front-end communication unit (10) further comprising a control unit (16) communicatively connected to the first communication interface (12), the control unit (16) being configured to transfer the first data (D1) to a back-end communication unit (18) and to transfer the second data (D2) via the first communication interface (12) to the implantable medical device (14); and
the back-end communication unit (18) comprising a programmer (20) configured to program the implantable medical device (14) through a real-time, bidirectional communication with the implantable medical device (14) via the control unit (16) and the first communication interface (12) of the front-end communication unit (10).

2. Implant communication system of claim 1, wherein the programmer (20) is configured to transmit a first control signal (22) and/or the first data (D1) to the control unit (16) of the front-end communication unit (10), wherein the control unit (16) transmits the first control signal (22) and/or the first data (D1) to the first communication interface (12), and wherein the first communication interface (12) transmits the first control signal (22) and/or the first data (D1) to the implantable medical device (14).

3. Implant communication system of claim 1 or 2, wherein the first communication interface (12) of the front-end communication unit (10) is configured to transmit a second control signal (24) and/or the second data (D2) read out from the implantable medical device (14) to the control unit (16) of the front-end communication unit (10), and wherein the control unit (16) is configured to transmit the second control signal (24) and/or the second data (D2) to the programmer (20).

4. Implant communication system of any one of the preceding claims, wherein the control unit (16) and the programmer (20) are configured to communicate through a peer-to-peer network connection or a client-server network connection, in which the control unit (16) comprises a client application and the programmer (20) comprises a server application.

5. Implant communication system of claim 4, wherein the programmer (20) is configured to initiate the network connection with the control unit (16) by inputting, in particular by a user of the programmer (20), a session key (26) generated by the control unit (16).

6. Implant communication system of claim 5, wherein the programmer (20) is configured to establish a connection with the control unit (16) via a cloud server (28).

7. Implant communication system of claim 6, wherein if the session key (26) input into the programmer (20) matches the session key (26) generated by the control unit (16), wherein the programmer (20) and the control unit (16) have a network connection to the cloud server (28), the programmer (20) and the control unit (16) are configured to establish an encrypted connection with each other via the cloud server (28).

8. Implant communication system of any one of the preceding claims, wherein the communications units (10, 18) are configured to establish a secure communication link between each other using the session key or another key.

9. Implant communication system of any one of the preceding claims, wherein the programmer (20) is a standalone medical programming device and/or an app on a smartphone or tablet computing device, said programmer (20) further having Bluetooth-, WLAN- und/or LAN-based connectivity.

10. Implant communication system of any one of the preceding claims, wherein if the network connection between the control unit (16) and the programmer (20) is established, the programmer (20) is configured to redirect the communication of the programmer (20) to the first communication interface (12) to a communication between the programmer (20) and the control unit (16).

11. Implant communication system of claim 10, wherein upon establishment of the communication between the programmer (20) and the control unit (16), the first communication interface (12) is placeable in proximity to the implantable medical device (14) in order to read out the first data (D1), in particular a model type, a firmware version and/or current setting data, in particular a current programming, from the implantable medical device (14) and/or to write the second data (D2) to the implantable medical device (14).

12. Implant communication system of any one of the preceding claims, wherein the control unit (16) of the front-end communication unit (10) is configured to check an integrity of the second data (D2) for the implantable medical device (14) and whether the second data (D2) is intended for the currently interrogated implantable medical device (14).

13. Implant communication system of claim 12, wherein if the integrity of the second data (D2) and a correctness of the currently interrogated implantable medical device (14) are confirmed, the first communication interface (12) is configured to transfer the second data (D2) to the implantable medical device (14), wherein after programming the implantable medical device (14), the front-end communication unit (10) is configured to send a confirmation message (34) to the programmer (20).

14. Computer-implemented method for communicating with an implantable medical device (14) comprising the steps of:
providing (S1) a front-end communication unit (10) comprising a first communication interface (12) configured to read out first data (D1) from an implantable medical device (14) and to write second data (D2) to the implantable medical device (14);
providing (S2) a control unit (16) comprised by the front-end communication unit (10), the control unit (16) being communicatively connected to the first communication interface (12);
transferring (S3) the first data (D1) to a back-end communication unit (18) by means of the control unit (16);
transferring (S4) the second data (D2) via the first communication interface (12) to the implantable medical device (14); and
programming (S5) the implantable medical device (14) through a real-time, bidirectional communication with the implantable medical device (14) via the control unit (16) and the first communication interface (12) of the front-end communication unit (10) by means of a programmer (20) comprised by the back-end communication unit (18).

15. Computer program with program code to perform the method of claim 14 when the computer program is executed on a computer.
